# EUROPEAN PATENT APPLICATION

(11) **EP 4 397 306 A1**
(43) Date of publication of application: **10.07.2024**
(21) Application number: 23150466.3
(22) Date of filing: 05.01.2023
(51) Int. Cl.: A61K 31/426, A61K 31/155, A61K 31/166, A61K 31/40, A61K 31/445, A61P 17/00, A61P 27/02, A61P 27/12, A61P 37/00, A61K 31/404, A61K 31/4453, A61K 39/395

(54) **C5AR1 INHIBITORS FOR USE IN THE TREATMENT OF OCULAR MUCOUS MEMBRANE PEMPHIGOID AND/OR ORAL MUCOUS MEMBRANE PEMPHIGOID**

(71) Applicant: Dompe' Farmaceutici S.P.A., 20122 Milan (IT)
(72) Inventor: SCHMIDT, Enno, 23538 Lübeck (DE); LUDWIG, Ralf, 23538 Lübeck (DE); PATZELT, Sabrina, 23538 Lübeck (DE); ARAMINI, Andrea, 67100 L'Aquila (IT); BRANDOLINI, Laura, 67100 L'Aquila (IT); COCCHIARO, Pasquale, 67100 L'Aquila (IT); BIANCHINI, Gianluca, 67100 L'Aquila (IT); DETTA, Nicola, 80131 Napoli (IT)
(74) Representative: Dompé farmaceutici Spa

(57) **Abstract**

The invention relates to C5aRl inhibitors for use in the prevention and/or treatment of ocular mucous membrane pemphigoid and/or oral mucous membrane pemphigoid.

## Description

### TECHNICAL FIELD

The invention relates to C5aR1 inhibitors useful in the prevention and/or treatment of ocular mucous membrane pemphigoid (OcMMP) and/or oral mucous membrane pemphigoid.

### BACKGROUND OF THE INVENTION

Mucous membrane pemphigoid (MMP) is a systemic cicatrizing autoimmune disease that primarily affects orificial mucous membranes, such as the conjunctiva, the nasal cavity, the oropharynx, and the genitalia.

About 75% of MMP patients generate antibodies against BP180 (type XVII collagen) and 25% of MMP patients against laminin 332. In less than 5% of MMP patients, antibodies against type VII collagen or α6β4 integrin are detected (Domloge-Hultsch, N., et al., J Clin Invest, 1992. 90(4): p. 1628-33; Oyama, N., et al., Br J Dermatol, 2006. 154(1): p. 90-8; Schmidt, E., et al., Br J Dermatol, 2001. 145(5): p. 778-83).

Ocular involvement occurs in about 70% of all MMP cases and ocular MMP is the leading cause of cicatrizing conjunctivitis in developed countries.

Linear immunoglobulin A disease, mucosal dominated epidermolysis bullosa acquisita, and anti-laminin 332/anti-epiligrin/anti-laminin 5 pemphigoid are encompassed by Ocular MMP (OcMMP).

The progressive inflammatory and scarring nature of ocular MMP leads to severe visual impairment in 30% of affected eyes and bilateral blindness in 20%.

Ocular MMP in often associated with oral mucosa lesions including desquamative gingivitis, vesicles, erosions covered by pseudomembranes and ulcers.

In some cases of MMP, only the oral mucosa is involved.

The underlying pathophysiological mechanism of the disease is a type 2 hypersensitivity reaction against the basal epithelial membrane of the conjunctiva.

In particular, conjunctival involvement is critical since the autoantibody-induced inflammation results in conjunctival scarring that may even progress after the inflammatory process has halted and leads to visual impairment and blindness.

Early diagnosis and appropriate treatment are of paramount importance to avoid inflammatory and infectious complications, as well as possible visual loss. Several mechanisms have been proposed underlying the development of antibody-mediated MMP.

Ocular MMP management is aimed at controlling the immune-mediated inflammatory disease preventing fibrosis and progression of the disease.

Georgoudis, P. et al, "Ocular Mucous Membrane Pemphigoid: Current State of Pathophysiology, Diagnostics and Treatment", Ophthalmol. Ther. (2019) 8, 5-17, discloses that a stepladder approach is used to select immunosuppressive agents and to escalate treatment, depending on disease severity (mild, moderate, severe). The medications used are dapsone, sulphapyridine, sulphasalazine, azathioprine (AZA), methotrexate (MTX), mycophenolate mofetil (MMF), cyclophosphamide, and short courses of oral steroids.

CD20 monoclonal antibodies, TNFα, inhibitors, and intravenous immunoglobulin (IVIg) are used to treat disease in patients non-responsive to conventional immunosuppressants.

The therapeutic mainstay are high-dose systemic corticosteroids supplemented with potentially corticosteroid sparing agents such as azathioprine, mycophenoles, dapsone, antibiotics with anti-inflammatory activity such as doxycycline, high-dose-intravenous immunoglobulins, and the anti-CD20 antibody rituximab.

The current treatment armamentarium is frequently not effective and associated with severe adverse events.

Thus, there is still a high need for effective and safe therapies.

Different C5aR1 inhibitors have been developed and are well known to the skilled man.

WO2007/060215 discloses (R)-arylalkylamino derivatives and their use in the treatment of diseases that involve C5a induced human PMNs chemotaxis such as sepsis, psoriasis, bullous pemphigoid, rheumatoid arthritis, ulcerative colitis, acute respiratory distress syndrome, idiopathic fibrosis, cystic fibrosis, chronic obstructive pulmonary disease, glomerulonephritis and in the prevention and the treatment of injury caused by ischemia and reperfusion.

It is therefore the aim of the invention to provide an effective treatment of MMP, in particular ocular and oral MMP.

### SUMMARY OF THE INVENTION

The invention is directed to C5aR1 inhibitors for use in the prevention and/or treatment of ocular mucous membrane pemphigoid (OcMMP) and/or oral mucous membrane pemphigoid in a subject.

The invention is also directed to pharmaceutical compositions comprising a C5aR1 inhibitor and at least one pharmaceutically acceptable excipient or carrier for use in the prevention and/or treatment of ocular mucous membrane pemphigoid and/or oral mucous membrane pemphigoid in a subject.

The invention is also directed to a method of preventing and/or treating ocular mucous membrane pemphigoid and/or oral mucous membrane pemphigoid, which comprises administering an effective amount of one or more C5aR1 inhibitor compounds of the invention to a subject in need thereof.

The invention is also directed to the use of the claimed C5aR1 inhibitors in the manufacture of a medicament for the prevention and/or treatment of ocular mucous membrane pemphigoid and/or oral mucous membrane pemphigoid in a subject.

### BRIEF DESCRIPTION OF FIGURES

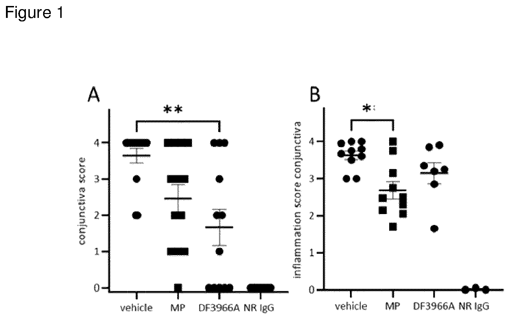

**Figure 1** shows the effects of treatment with DF3966A, administered by topical ophthalmic application, or methylprednisolone (MP), administered by intraperitoneal injection, in an experimental MMP mouse model at day 12 after the initial anti-mLAMα3 IgG injection, compared to control treatment with vehicle, by topical ophthalmic administration (vehicle). In details:
   Panel A shows the effect of DF3966A or MP on palpebral conjunctival split formation at day 12, expressed as conjunctiva score. Data are presented as means +/- standard deviation. On day 12 there is a statistically significant difference between the vehicle group and DF3966A (p=0.0016). Animals treated with IgG isolated from normal rabbit serum injected s.c. (NR IgG), were used as negative control.
   Data are based on 12-14 mice per group, except for NR IgG (n=10). The asterix indicates statistical significance (ANOVA with Holm Sidaks method for multiple comparisons).
   Panel B shows semi-quantification of subepithelial inflammatory infiltrate based on hematoxylin and eosin (H&E) stained biopsies of the palpebral conjunctiva, in mice treated with of DF3966A or MP. On day 12 there is a statistically significant difference between the vehicle group and MP group. Animals treated with IgG isolated from normal rabbit serum injected s.c. (NR IgG), were used as negative control.
   Data are presented as means +/- standard deviation. Data are based on 7-10 mice per group, except for NR IgG (n=3). The asterix indicates statistical significance (ANOVA with Holm Sidaks method for multiple comparisons).

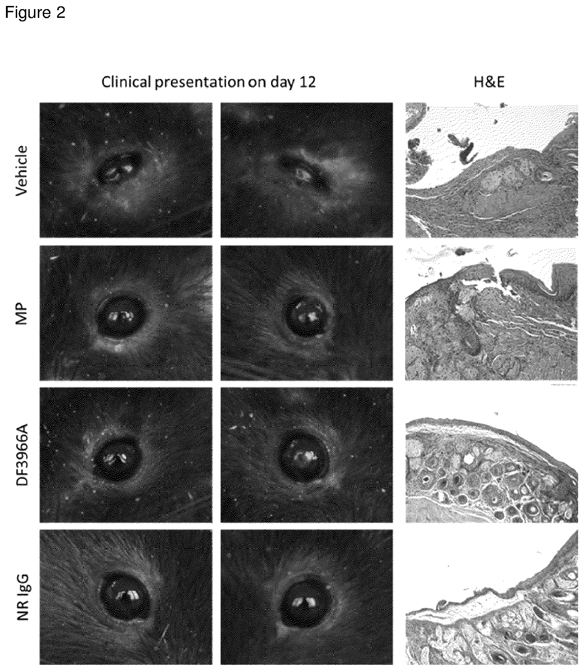

**Figure 2** shows representative clinical presentations of the eyes and H&E stained sections of the palpebral conjunctiva obtained 12 days after the initial anti-mLAMα3 IgG injection, in mice treated with DF3966A, by topical ophthalmic administration, or methylprednisolone (MP), by intraperitoneal injection.

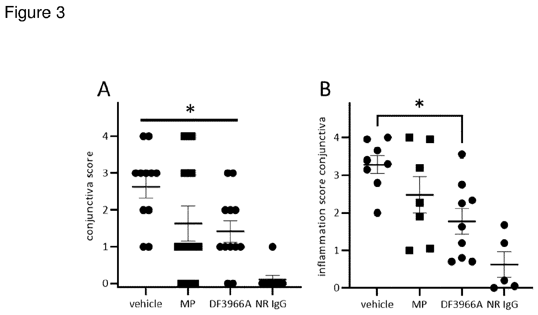

**Figure 3** shows the reduction of split formation in the palpebral conjunctiva in experimental MMP mice at day 28 after the initial anti-mLAMα3 IgG injection, treated with DF3966A, by topical ophthalmic administration, or methylprednisolone (MP), by intraperitoneal injection. In details:
   Panel A shows the effect of treatment with DF3966A or MP on palpebral conjunctival split formation on day 28, expressed as conjunctiva score, compared to vehicle. Animals treated with IgG isolated from normal rabbit serum injected s.c. (NR IgG) were used as negative control.
   Data are presented as means +/- standard deviation. On day 28 there is a statistically significant difference between the vehicle group and DF3966A (p=0.0414). Data are based on 11-14 mice per group, except for NR IgG (n=9). The asterix indicates statistical significance (ANOVA with Holm Sidaks method for multiple comparisons).
   Panel B shows the semi-quantification of subepithelial inflammatory infiltrate based on hematoxylin and eosin (H&E) stained biopsies of the palpebral conjunctiva in mice treated with DF3966A or MP, compared to vehicle. Animals treated with IgG isolated from normal rabbit serum injected s.c. (NR IgG) were used as negative control. Data are presented as means +/- standard deviation. On day 28 there is a statistically significant difference between the vehicle group and DF3966A (p=0.0188). Data are based on 7-9 mice per group, except for NR IgG (n=5). The asterix indicates statistical significance based on ANOVA with Dunnetts method for multiple comparisons.

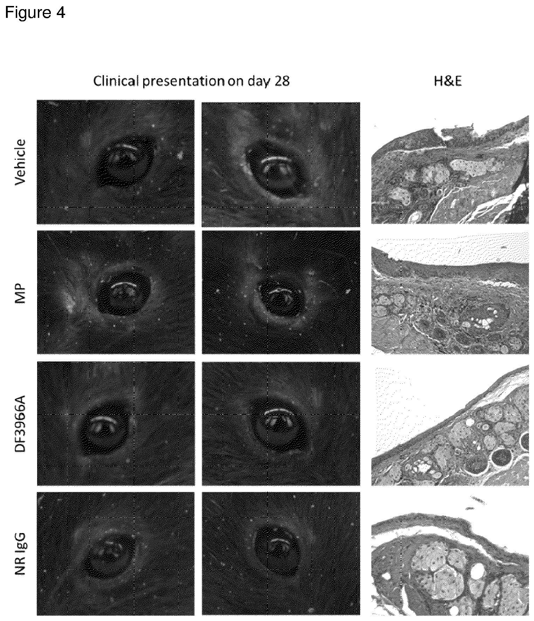

**Figure 4** shows representative clinical presentations of the eyes and H&E stained sections of the palpebral conjunctiva obtained 28 days after the initial anti-mLAMα3 IgG injection, in mice treated with DF3966A, by topical ophthalmic administration, or methylprednisolone (MP), by intraperitoneal injection.

### DETAILED DESCRIPTION OF THE INVENTION

It has been surprisingly found that the C5aR1 inhibitors are effective in the prevention and/or treatment of ocular mucous membrane pemphigoid (OcMMP) and/or oral mucous membrane pemphigoid in a subject.

The terms "treatment" and "prevention" as used herein refer to the eradication/amelioration or prevention/delay in onset, respectively, of the disorder being treated or of one or more of the symptoms associated thereof, notwithstanding the fact that the patient may still be afflicted with the underlying disorder.

Accordingly, the invention is directed to C5aR1 inhibitors for use in the prevention and/or treatment of ocular mucous membrane pemphigoid and/or oral mucous membrane pemphigoid.

The term "C5aR1 inhibitor" in accordance with the present invention means any compound that interacts with C5aR1 and prevents its binding to C5a or that blocks the signaling of C5aR1 upon binding of C5a.

Preferably, said C5aR1 inhibitor compound is selected from C5aR1 competitive antagonists, anti-C5aR1 antibodies able to block the C5a binding sites on the receptor and C5aR1 non-competitive allosteric inhibitors, more preferably said C5aR1 inhibitor compound is a non-competitive allosteric inhibitor.

"Noncompetitive allosteric inhibitor of C5a receptor" according to the present invention means a compound that interacts with C5a receptors in an allosteric site, located in the TM region, and inhibits intracellular signal transduction events activated by the agonist binding, without affecting any binding of endogen ligand C5a on its receptor.

According to one preferred embodiment, said C5aR1 competitive antagonists are selected from the group consisting of:
- (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide (Avacopan, Vynpenta^{®});
- N-Acetyl-L-phenylalanyl-L-ornithyl-L-prolyl-3-cyclohexyl-D-alanyl-L-tryptophyl-L-arginine-N-5.2-C-1.6-lactam (PMX-53);
- Acetylated phenylalanine-[ornithyl-proline-(D)cyclohexylalanine-tryptophyl-arginine];
- L-Alanyl-L-seryl-glycyl-L-alanyl-L-prolyl-L-alanyl-L-prolyl-glycyl-L-prolyl-L-alanyl-glycyl-L-prolyl-L-leucyl-L-arginyl-L-prolyl-L-methionyl-L-phenylalanine;
- N,N-Bis(1,3-benzodioxol-5-ylmethyl)-N-(1-butyl-2,4-diphenyl-1H-imidazol-5-ylmethyl)amine;
- N-[2-(4-Chlorophenyl)ethyl]-N-(1,4-dioxaspiro[4.5]dec-8-yl)-2-isobutylbenzamide;
- N-[2-(4-Chlorophenyl)ethyl]-N-(4-hydroxycyclohexyl)-1-benzothiophene-3-carboxamide;
- N-[2-(4-Chlorophenyl)ethyl]-N-(4-hydroxycyclohexyl)naphthalene-1-carboxamide;
- 2-(2-Ethyl-6-methylphenyl)-4-methoxy-N-(5-methoxy-2-methylphenyl)-5,6,7,8-tetrahydroquinolin-5-amine;
- 2-(2,6-Diethylphenyl)-N-ethyl-4-methoxy-N-(1-naphthyl)-5,6,7,8-tetrahydroquinolin-5-amine;
- N-[2,6-Dioxohexahydropyrimidin-4(S)-ylcarbonyl]-L-phenylalanyl-L-ornithyl-L-prolyl-5-methyl-L-norleucyl-4-fluoro-L-phenylalanyl-L-phenylalaninamide (JPE-1375; JSM-1375);
- N-(3-Phenylpropionyl)-L-ornithyl-L-prolyl-3-cyclohexyl-D-alanyl-L-tryptophyl-L-arginine N-5.1-C-1.5-lactam (PMX-205);
- N,N'-Bis(4-amino-2-methylquinolin-6-yl)urea (NSC12155);
- N-[4-(Dimethylamino)benzyl]-N-(4-isopropylphenyl)-7-methoxy-1,2,3,4-tetrahydronaphthalene-1-carboxamide hydrochloride (W54011);
- L-Phenylalanyl-L-ornithyl-L-prolyl-3-cyclohexyl-D-alanyl-L-tryptophyl-L-arginine N-5.2-C-1.6-cyclic peptide;
- (4aR, 16aS)-6, 18- Dihydroxy-23(S)-[2(S)-hydroxy-2-[2(R)-hydroxy-6(R)-methyl-5(R)-[2(S)-methylbutyl]tetrahydro-2H-pyran-2-yl]propionamido]-22(S)-isopropyl-7(S), 19(R)-dimethyldocosahydro-13H,22H -dipyridazino[6, 1-f:6',1'o][1,4,7,10,13,16]oxapentaazacyclononadecine-5,7,11,17,20,24-hexanone (L-156602).

Anti-C5aR1 antibodies are commercially available, for example they are sold by abcam, ThermoFisher Scientific, Biocompare.

According to one preferred embodiment, the anti-C5aR1 antibodies or aptamers are selected from the group consisting of:
- Avdoralimab (IPH-5401)
- MOR-044254, also known as anti-C5aR monoclonal antibody TJ210;
- NOX-D20, a PEGylated biostable mirror-image mixed (L)RNA/DNA aptamer (Spiegelmer);
- Anti-C5aR1ab-C5-SiRNA conjugate, anti-C5aRlab-protamine-C5siRNA conjugate;
- m20/70 mlgG2a.1;
- 3C5;
- 7F3.

According to one preferred embodiment, said non-competitive allosteric inhibitors are selected from:
(2R)-2-[3-(furan-2-carbonyl)phenyl]-N-[4-(trifluoromethyl)-1,3-thiazol-2-yl]propenamide (DF2427);
and
compounds having general formula (I): 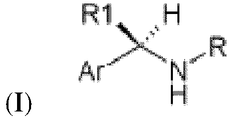 or a pharmaceutically acceptable salt thereof, wherein
   **R** is selected from:
      - 2-thiazolyl or 2-oxazolyl, unsubstituted or substituted by a group selected from methyl, tert-butyl or trifluoromethyl group;
      - C(Ra)=N-W wherein W is linear or branched C₁-C₄ alkyl,
      - CORa, SORa, SO₂Ra, PORa, PO₂Ra,
   wherein **Ra** is selected from:
      - C₁-C₅-alkyl, C₃-C₆-cycloalkyl, C₂-C₅-alkenyl, unsubstituted or substituted phenyl with a group selected from halogen, C₁-C₄-alkyl, C₁-C₄-alkoxy, halo-C₁-C₄-alkoxy, hydroxy, C₁-C₄-acyloxy, phenoxy, cyano, nitro, amino;
      - a heteroaryl group selected from pyridine, pyrimidine, pyrrole, thiophene, furane, indole, thiazole, oxazole, such heteroaryl being unsubstituted or substituted with a group selected from halogen, C₁-C₄-alkyl, C₁-C₄-alkoxy, halo-C₁-C₄-alkoxy, hydroxy, C₁-C₄- acyloxy, phenoxy, cyano, nitro, amino;
      - a α or β carboxyalkyl residue consisting of straight or branched C₁- C₆-alkyl, C₃-C₆-cycloalkyl, C₂-C₆-alkenyl, C₁-C₆-phenylalkyl, optionally substituted with a further carboxy (COOH) group;
      - an ω-aminoalkylamino group of formula (II):
         wherein in said 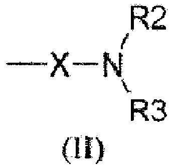 formula (II) X is selected from:
         - linear or branched C₁-C₆ alkylene, C₄-C₆ alkenylene, C₄-C₆ alkynylene, optionally substituted by:
            a) a CO₂R4 group, wherein R4 represents hydrogen or a linear or branched C₁-C₆ alkyl group or a linear or branched C₂-C₆ alkenyl group, or
            b) a CONHR5 group wherein R5 represents
         - hydrogen, linear or branched C₂-C₆ alkyl or an OR4 group, R4 being defined as above;
         - a (CH₂)ₘ-B-(CH₂)ₙ, group, optionally substituted by a CO₂R4 or CONHR5 group, as defined above, wherein B is an oxygen, or sulfur atom, or nitrogen atom optionally substituted by a C₁-C₄ alkyl group, m is zero or an integer from 2 to 3 and n is an integer from 2 to 3; or B is a CO, SO or CONH group, m is an integer from 1 to 3 and n is an integer from 2 to 3;
   **R2** and **R3** are independently selected form hydrogen, linear or branched C₁-C₆ alkyl, optionally interrupted by an oxygen or sulfur atom, a C₃-C₇ cycloalkyl, C₃-C₆ alkenyl, C₃-C₆-alkynyl, aryl-C₁-C₃-alkyl, hydroxy-C₂-C₃-alkyl group; or
   **R2** and **R3** together with the N atom to which they are bound, form a 3-7 membered nitrogen heterocyclic ring of formula (III) 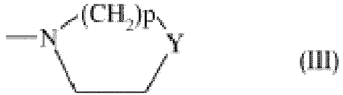 wherein Y represents:
      - a single bond, CH₂, O, S, or a N-R6 group, where R6 represents hydrogen, C₁-C₄ alkyl, C₁-C₄ acyl, unsubstituted or substituted phenyl with a group selected from halogen, C₁-C₄-alkyl, C₁-C₄- alkoxy, hydroxy, C₁-C₄-acyloxy, phenoxy, cyano, nitro, amino, and p represents an integer from 0 to 3;
      - a residue of formula SO₂R7 wherein R7 is C₁-C₆-alkyl, C₃-C₆-cycloalkyl, C₂-C₆-alkenyl, aryl and heteroaryl;
      or
      in said formula (II), **X** together with the nitrogen atom to which it is bound and with the **R2** group, forms a nitrogen containing 3-7 membered heterocyclic, monocyclic or polycyclic ring, and **R3** is selected from the group comprising hydrogen, C₁-C₄ alkyl, C₁-C₄ acyl, unsubstituted or substituted phenyl with a group selected from halogen, C₁-C₄-alkyl, C₁-C₄-alkoxy, hydroxy, C₁-C₄- acyloxy, phenoxy, cyano, nitro, amino;
   **R1** is linear or branched C₁-C₅ alkyl, C₃-C₅ cycloalkyl; **Ar** is selected from:
      - a phenyl group unsubstituted or substituted by one or more groups independently selected from halogen, C₁-C₄-alkyl, C₁-C₄-alkoxy, hydroxy, C₁-C₄-acyloxy, phenoxy, cyano, nitro, amino, C₁-C₄-acylamino, halo-C₁- C₃- alkyl, halo-C₁-C₃-alkoxy, benzoyl, heteroaryl carbonyl, heteroaryl, linear or branched C₁-C₈-alkanesulfonate, linear or branched C₁-C₈-alkanesulfonamides, linear or branched C₁-C₈ alkyl sulfonylmethyl; or
      - a heteroaryl ring selected from pyridine, pyrrole, thiophene, furan, indole.

Among the above compounds, particularly preferred are compounds of said formula (I) or pharmaceutically acceptable salts thereof, wherein:
**R** is selected from:
   - 2-thiazolyl or 2-oxazolyl, unsubstituted or substituted by a group selected from methyl, tert-butyl or trifluoromethyl group,
   - C(Ra)=N-W wherein W is linear or branched C₁-C₄ alkyl,
   - CORa, SORa or SO₂Ra,
wherein Ra is as defined above; and
**Ar** is selected from the group comprising:
3'-benzoylphenyl, 3'-(4-chloro-benzoyl)-phenyl, 3'-(4-methylbenzoyl)-phenyl, 3'-acetyl-phenyl, 3'-propionyl-phenyl, 3'-isobutanoyl-phenyl, 4'-isobutyl-phenyl, 4'-trifluoromethanesulfonyloxy-phenyl, 4'-benzenesulfonyloxy-phenyl, 4'-trifluoromethanesulfonylamino-phenyl, 4'-benzenesulfonylamino-phenyl, 4'-benzenesulfonylmethyl-phenyl, 4'-acetoxyphenyl, 4'-propionyloxy-phenyl, 4'-benzoyloxy-phenyl, 4'-acetylamino-phenyl, 4'-propionylamino-phenyl, 4'- benzoylamino-phenyl, 3' -(furan-2-carbonyl)-phenyl, 3'-(benzofuran-2-carbonyl)-phenyl, 3' -(thiophen-2-carbonyl)-phenyl, 3' -(pyridine-2-carbonyl)-phenyl, 3 '-(thiazole-2-carbonyl)-phenyl, 3'-(oxazole-2-carbonyl)-phenyl, 3'-(2-furyl)-phenyl, 3'-(2-oxazolyl)- phenyl, 3'-(3-isoxazolyl)-phenyl, 3'-(2-benzoxazolyl)-phenyl, 3'-(3- benzoisoxazolyl)-phenyl, 3'-(2-thiazolyl)-phenyl, 3'-(2-pyridyl)-phenyl, 3'-(2- thiophenyl)-phenyl;
or **Ar** is a heteroaryl ring selected from pyridine, pyrrole, thiophene, furan or indole.

According to a further embodiment, (R)-arylalkylamino derivatives are compounds of formula (I) as defined above, wherein:
R is
- 2-thiazolyl, unsubstituted or substituted by a group selected from methyl or trifluoromethyl;
- CORa, SO2Ra,
wherein Ra is selected from:
- C₁-C₅-alkyl, Cs-Cs-cycloalkyl;
- phenyl, 2-pyridyl, 2-furyl, 2-thiophenyl groups;
- a group of formula II,
   wherein
   X represents: 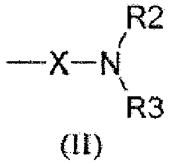
   linear or branched C₁-C₆ alkylene,
   R2 and R3 together with the N atom to which they are bound, form a 4-6 membered nitrogen containing heterocyclic ring of formula (III) 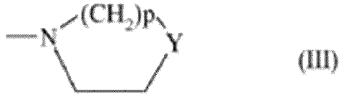
   wherein Y represents CH₂ and p represents an integer from 0 to 2;
   R1 is methyl;
   Ar is selected from:
      3' -benzoylphenyl, 3' -(4-chloro-benzoyl)-phenyl, 3' -(4-methyl-benzoyl)-phenyl,
      4'-trifluoromethanesulfonyloxy-phenyl, 4'-benzenesulfonyloxy-phenyl, 3'-(furan-2-carbonyl)-phenyl.

Preferred compounds of formula (I) according to the invention are selected from:
- 4-{(1R)-1-[(phenylsulfonyl)amino]ethyl}phenyl trifluoromethanesulfonate;
- N-[(1R)-1-(3-benzoylphenyl)ethyl]benzenesulfonamide;
- 4-{(1R)-1-[(pyridine-3-ylsulfonyl)amino]ethyl}phenyltrifluoromethanesulfonate;
- N-[(1R)-1-(3-benzoylphenyl)ethyl]methanesulfonamide;
- N-{(1R)-1-[3-(2-furoyl)phenyl]ethyl}thiophene-2-sulfonamide;
- N-{(1R)-1-[3-(2-furoyl)phenyl]ethyl}methanesulfonamide;
- 4-{(1R)-1-[(thien-2-ylsulfonyl)amino]ethyl}phenyl trifluoromethanesulfonate;
- N-[(1R)-1 -(3 -benzoylphenyl)ethyl] thiophene-2-sulfonamide;
- N-[(1R)-1-(3-benzoylphenyl)ethyl]-3-pyrrolidin-1-ylpropane-1-sulfonamide;
- methyl 5-({[(1R)-1-(3-benzoylphenyl)ethyl]amino}sulfonyl)-2-furoate;
- 5-({[(1R)-1-(3-benzoylphenyl)ethyl]amino}sulfonyl)-2-furoicacid;
- 4-{(1R)-2-methyl-1-[(methylsulfonyl)amino]propyl}phenyltrifluoromethanesulfonate;
- N-((1R)-1-{4-[1-methyl-1-(phenylsulfonyl)ethyl]phenyl } ethyl)methanesulfonamide;
- 4-[(1R)-1-(isobutyrylamino)ethyl]phenyltrifluoromethanesulfonate;
- 4-{[(1R)-1-(pyridine-3-ylcarbonyl)amino]ethyl]}phenyltrifluoromethanesulfonate;
- N-[(1R)-1-(3-benzoylphenyl)ethyl]benzamide;
- N-[(1R)-1-(3-benzoylphenyl)ethyl]-2-furamide;
- N-[(1R)-1-(3-benzoylphenyl)ethyl]cyclobutanecarboxamide;
- N-[(1R)-1-(4-trifluoromethanesulfonyloxy)phenylethyl]-4-piperidin-1-yl butanamide (DF2593Y);
- 4-{(1R)-1-[(4-pyrrolidin-1-ylbutanoyl)amino]ethyl]}phenyl trifluoromethanesulfonate;
- 3-{(1R)-1-[4-(4-trifluoromethyl-1,3-thiazol-2-yl)amino]ethyl}phenyl) (phenyl)methanone;
- R(-)-2-[(4'-trifluoromethanesulfonyloxy)phenyl]-N-[3-(N'-pirrolidinyl)propyl] propionamide (DF2297X) or its chloride salt (DF2297A); and
- 5-[(1R)-1-(4-{[4-(trifluoromethyl)-1,3-thiazol-2-yl]amino}phenyl)ethyl]tetrazol-2-ide (DF3966Y) or its sodium salt (DF3966A).

Particularly preferred compounds of formula (I) according to the invention are selected from:
- N-[(1R)-1-(4-trifluoromethanesulfonyloxy)phenylethyl]-4-piperidin-1-yl butanamide (DF2593Y) and pharmaceutically acceptable salts thereof, preferably its chloride salt (DF2593A),
- R(-)-2-[(4'-trifluoromethanesulfonyloxy)phenyl]-N-[3-(N'-pirrolidinyl)propyl] propionamide (DF2297X) or its chloride salt (DF2297A), and
- 5-[(1*R*)-1-(4-{[4-(trifluoromethyl)-1,3-thiazol-2-yl]amino}phenyl)ethyl]tetrazol-2-ide (DF3966Y) or its sodium salt (DF3966A).

Compounds of formula (I) are described in WO2007/060215, which also discloses their method of synthesis.

Preferred C5aR1 inhibitors according to the invention are Avacopan, PMX-53, W54011, Avdoralimab, MOR-044254, PMX-205, DF2593A, DF2297A, DF2427 and DF3966A, more preferably the C5aR1 inhibitor is DF3966A. Preferably, the C5aR1 inhibitor for use according to the present invention is administered topically to the surface of the eye of a subject. Preferably, the C5aR1 inhibitor for use according to the present invention is formulated in form of eyedrops.

As will be discussed in the experimental section, the present inventors have shown that topical application of a C5aR1 inhibitor the surface of the eye results in amelioration of both ocular and oral MMP.

The invention is also directed to a pharmaceutical composition comprising a C5aR1 inhibitor, as above described, and at least one pharmaceutically acceptable excipient or carrier for use in the prevention and/or treatment of ocular mucous membrane pemphigoid and/or oral mucous membrane pemphigoid.

Preferably, the pharmaceutical composition is an ophthalmic composition suitable for topical application to the surface of the eye.

Accordingly, the invention is further directed to an ophthalmic composition comprising a therapeutically effective amount of a C5aR1 inhibitor, as above described, and at least one ophthalmologically acceptable excipient or carrier. An "ophthalmologically acceptable excipient" is an inert excipient which allows delivery of a medicament to the eye and/or eyelids, to treat an ocular disease or condition without deleterious effects on the eye.

According to an embodiment, said ophthalmic composition may be a liquid, eye drop composition for topical administration to the anterior segment of the eye. Said liquid composition may be in form of a solution, emulsion, or suspension. Said liquid composition may include micelles.

In one embodiment, the liquid composition is an aqueous composition.

Preferably, the liquid composition is an aqueous eye drop composition.

Preferably, said liquid composition comprises ophthalmologically acceptable excipients selected from ophthalmologically acceptable viscosity enhancers, penetration enhancers, buffering agents, osmolarity regulators, preservatives and surfactants.

Viscosity enhancers have the function to increase viscosity of the composition and to improve its retention in the conjunctival sac and are preferably selected from cellulose derivatives, preferably hydroxymethylcellulose, hydroxyethylcellulose, hydroxypropylmethylcellulose, methylcellulose; polyvinylpyrrolidone and gelling agents, preferably gellan, xanthan gum and carbopol-974.

Penetration enhancers have the function of enhancing drug permeability across ocular membranes and are preferably selected from cyclodextrins, chelating agent, crown ethers, bile acids and bile salts.

Buffering agents have the function of providing and maintaining the correct pH of the formulation to be compatible for use in the eye, preferably at a pH comprised between 6 and 8. The preferred buffer is phosphate buffer, but other buffers capable of maintaining the pH within the desired range, especially buffers suitable for ophthalmic use, are also included.

Osmolarity regulators are salts able to make the liquid composition isotonic with ocular fluids. The preferred salt is sodium chloride (NaCl) but other biologically acceptable salts may be used, such as for instance potassium chloride (KCl), calcium chloride (CaCl₂) and magnesium chloride (MgCl₂) and their admixtures.

Preservatives inhibit microbial activity. Suitable preservatives include for instance quaternary ammonium compounds such as benzalkonium chloride, cetyltrimethylammonium bromide and cetylpyridinium chloride.

Surfactants have the function of making the composition stable and are preferably selected from polysorbates such as Tween 80, poloxamers such as Pluronics F68 or proteins such as serum albumin.

Said liquid, eye drop composition can be part of a kit comprising the composition, a container for holding the composition and a drop dispenser.

A "therapeutically effective amount" according to the present invention means an amount sufficient to achieve treatment or prevention of the disease. Determination of the effective amounts is well within the capability of those skilled in the art based upon the achievement of a desired effect. An effective amount will depend on factors including, but not limited to, the weight of a subject and/or the degree of the disease or unwanted condition from which a subject suffers.

The invention relates also to the use of the above C5aR1 inhibitors in the manufacture of a medicament for the prevention and/or treatment of ocular mucous membrane pemphigoid and/or oral mucous membrane pemphigoid. The invention is also directed to a method of preventing and/or treating ocular mucous membrane pemphigoid and/or oral mucous membrane pemphigoid, which comprises administering an effective amount of one or more C5aR1 inhibitor of the invention to a subject in need thereof.

The invention is further illustrated by the following example.

### EXAMPLE

A study was designed to investigate and show the effect of pharmacological inhibition of C5aR exerted by the compound DF3966A in a mouse model of MMP.

### Materials and methods

### Test compounds

- DF3966A Sodium Salt (Lot. LC446-81-1 GLB21001, Dompé S.p.A.)
- Vehicle (solvent, Dompé S.p.A.) - (positive control)
- Methylprednisolone (MP) (sourced by UKSH, Lübeck - Supplier Sanofi) -

### (reference treatment)

### Animal model - Mice

Adult C57Bl/6 (B6) mice (male and female) aged at least 6 weeks old were used. Animals were maintained on a 12-h light-dark cycle at the animal facility of the University of Lübeck. Mice were held under SPF conditions and fed acidified drinking water and standard chow ad libitum. Protocols were approved by the Animal Rights Commission of the Ministry of Agriculture and Environment, Schleswig-Holstein.

### Generation, isolation and characterization of anti-mLAMα3 IgG

For production of anti-mLAMα3 IgG, New Zealand White rabbits were immunized subcutaneously with 250µg of an equimolar mixture of the 2 purified recombinant proteins (aa1656-1985 and aa2756-3330 of murine Laminin alpha3 chain, produced in E. coli as disclosed by Heppe, E.N., et al., J Invest Dermatol, 2017, 137, 1709-1718) suspended in complete Freund's adjuvant. The animals were boosted twice with the same protein preparation in incomplete Freund's adjuvant. Immune sera were obtained at regular intervals and characterized by IF microscopy on cryosections of murine skin. IgG from rabbits immunized with recombinant fragments of murine mLAMα3 and from non-immunized rabbits was purified by affinity chromatography using protein G sepharose affinity chromatography (Amersham Biosciences, Heidelberg, Germany). Reactivity of IgG fractions was analyzed by IF microscopy on murine skin (Sitaru et al., Induction of complement fixing autoantibodies against type VII collagen results in subepidermal blistering in mice. J Immunol 2006, 177: 3461-8). In addition, each batch of anti-mLAMα3 IgG was characterized *in vivo* regarding its capability to induce experimental MMP in C57Bl/6 (B6) mice (WP1.1). Form this experiment, the amount of antibodies was determined, that leads to moderate experimental MMP; i.e. the amount needed to induce disease development with moderate involvement of the conjunctiva (i.e. score of 1-3 in a maximum of 50% of the animals on day 12) in the model of antibody transfer-induced MMP. For this purpose, the intensity of the separation of the conjunctiva is determined histologically. The body surface area affected should be between 3-8% at the concentration used. The conjunctiva score was set histologically post-mortem and was in a range of 0-4, depending on the intensity of the separation of the conjunctiva. The amount needed was determined to be 5 mg/injection of anti-mLAMα3 IgG.

### Study design

### Induction of experimental MMP by repetitive injections of anti-mLAMα3 IgG and treatment protocol

To test DF3966A at 0.05% effects on the severity of conjunctival involvement in experimental MMP, the disease was induced by repetitive subcutaneous (s.c.) injections of anti-mLAMα3 IgG (5mg/mouse) on alternating days (0, 2, (...) and 10) into adult B6 mice, which were treated throughout the entire experiment twice a day via eyedrops. Three eyedrops per eye were given twice daily. Mice treated with solvent via eyedrops or methylprednisolone (MP) via i.p. injections were used as treatment controls. Mice s.c. injected with IgG isolated from normal rabbit serum (NR IgG) served as negative control.

### Evaluation of conjunctival lesions

The primary endpoint of this experiment was the extent of conjunctival lesions as determined by lesional histopathology (H&E stain) on day 12 and 28 following an established scoring system (Heppe, E.N., et al., J Invest Dermatol, 2017, 137, 1709-1718). In detail, biopsies from the palpebral conjunctiva were taken on day 12 and day 28 and paraffine embedded. 4.5µm thick sections from 3 different depths of the biopsies were cut in triplicate and H&E stained for further quantification. Among the H&E stained histologies those with a palpebral conjunctival-epithelium with more than 1000µm were used for quantification. For this score the length of the split formation (split = epithelia separation from the underlying dermal structures) is measured. The split length is categorized to a score of 0-4 with no split = 0, less than 100µm = 1, less than 200µm = 2, less than 300µm = 3, more or equal 300µm = 4. Splits that occurred at the end of the tissue were excluded, because they were most likely to be of artificial nature due to the cutting. The longest split out of 9 possible sections is decisive for the final score.

The endpoint was analyzed on day 12 in the following groups:
▪ normal rabbit IgG (n=10)
▪ Anti-mLAMα3 IgG + solvent (positive control) (n=14)
▪ Anti-mLAMα3 IgG + MP (reference treatment) (n=13)
▪ Anti-mLAMα3 IgG + DF3966A (n=12)

The endpoint was analyzed on day 28 in the following groups:
▪ normal rabbit IgG (n=9)
▪ Anti-mLAMα3 IgG + solvent (positive control) (n=11)
▪ Anti-mLAMα3 IgG + MP (reference treatment) (n=11)
▪ Anti-mLAMα3 IgG + DF3966A (n=12)

The experiments were performed at two independent time points for each day 12 and day 28, including 7 mice/group and 5 mice/normal rabbit IgG.

### Evaluation of severity of oral lesions

The extent of oral lesions was determined by endoscopy (Videomed, München, Germany) at day 12 and 28, following an established scoring system. In detail, each affected mouth-quarter of the mouse is counted as one point if there are lesions/blisters/crusts/erosions. The quarters are defined as: left buccal mucosa, right buccal mucosa, hypopharynx, tongue. The maximum score is 4.

### Statistical analysis

For statistical analysis GraphPad Prism (version 8.4.3) was used. For comparison of treatment effects in multiple groups ANOVA was used. To isolate the group or groups that differ from others, Dunnett's or Holm Sidaks multiple comparison were used when appropriate.

### Results

### 1. Local application of DF3966A reduced split formation in the palpebral conjunctiva in experimental MMP

Injection of rabbit anti-mLAMα3 IgG into adult B6 mice persistently lead to induction of experimental MMP within 4-8 days after the first IgG injection.

After 12 days of twice daily local application of the drug as eyedrops a significant reduction of the conjunctival split formation for the group treated with DF3966A was observed, compared to mice that received the sole vehicle (**figure 1A**). No statistical significant reduction was seen in the subepithelial inflammatory cell infiltrate by semi-quantification of H&E stained palpebral conjunctival biopsies taken on day 12 of mice treated with DF3966A compared to those biopsies of mice that received the vehicle (**figure 1B**). In mice injected with NR-IgG no conjunctival lesions were observed compared to the other groups on day 12 (**figure 2**).

Treatment over 28 days twice daily via eyedrops lead to a significant reduction of palpebral conjunctival split formation in mice receiving DF3966A (**figure 3A**). This is also reflected in a decreased subepithelial inflammatory infiltrate by semi-quantification of H&E stained biopsies of the palpebral conjunctiva (**figure 3B**). Mice that just received NR-IgG from healthy rabbits did not show conjunctival lesions compared to the other groups on day 28 (**figure 4**). DF3966A reduced the conjunctival split formation on both 12 and 28 experimental days and decreased the inflammatory infiltrate after 28 days of treatment, compared to mice that received the solvent.

### 2. Local application of DF3966A reduces oral lesions in experimental MMP

Treatment with DF3966A decreased severity of oral involvement after 12 and 28 days of eyedrops application, with a statistically significant difference between the vehicle group and DF3966A (day 12: p=0.0479; day 28: p=0.0372).

### Conclusions

Overall, treatment with DF3966A twice daily via eyedrops reduced conjunctival split formation already on day 12 without reducing the underlying inflammation in these tissues. A longer lasting treatment until day 28 via eyedrops also decreased the inflammatory infiltrate in the palpebral conjunctiva. Furthermore, at both day 12 and 28 significant decrease in severity of oral lesions is observed.

## Claims

1. A C5aR1 inhibitor for use in the prevention and/or treatment of ocular mucous membrane pemphigoid and/or oral mucous membrane pemphigoid in a subject.

2. A C5aR1 inhibitor for use as claimed in claim 1, wherein said C5aR1 inhibitor is administered topically to the surface of the eye of the subject,

3. A C5aR1 inhibitor for use according to claim 1 or claim 2, selected from C5aR1 competitive antagonists, anti-C5aR1 antibodies, and C5aR1 non-competitive allosteric inhibitors.

4. A C5aR1 inhibitor for use according to any one of claims 1 to 3, wherein said inhibitor is a C5aR1 competitive antagonist selected from the group consisting of:
- (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide (Avacopan, Vynpenta^{®});
- N-Acetyl-L-phenylalanyl-L-ornithyl-L-prolyl-3-cyclohexyl-D-alanyl-L-tryptophyl-L-arginine-N-5.2-C-1.6-lactam (PMX-53);
- Acetylated phenylalanine-[ornithyl-proline-(D)cyclohexylalanine-tryptophyl-arginine];
- L-Alanyl-L-seryl-glycyl-L-alanyl-L-prolyl-L-alanyl-L-prolyl-glycyl-L-prolyl-L-alanyl-glycyl-L-prolyl-L-leucyl-L-arginyl-L-prolyl-L-methionyl-L-phenylalanine;
- N,N-Bis(1,3-benzodioxol-5-ylmethyl)-N-(1-butyl-2,4-diphenyl-1H-imidazol-5-ylmethyl)amine;
- N-[2-(4-Chlorophenyl)ethyl]-N-(1,4-dioxaspiro[4.5]dec-8-yl)-2-isobutylbenzamide;
- N-[2-(4-Chlorophenyl)ethyl]-N-(4-hydroxycyclohexyl)-1-benzothiophene-3-carboxamide;
- N-[2-(4-Chlorophenyl)ethyl]-N-(4-hydroxycyclohexyl)naphthalene-1-carboxamide;
- 2-(2-Ethyl-6-methylphenyl)-4-methoxy-N-(5-methoxy-2-methylphenyl)-5,6,7,8-tetrahydroquinolin-5-amine;
- 2-(2,6-Diethylphenyl)-N-ethyl-4-methoxy-N-(1-naphthyl)-5,6,7,8-tetrahydroquinolin-5-amine;
- N-[2,6-Dioxohexahydropyrimidin-4(S)-ylcarbonyl]-L-phenylalanyl-L-ornithyl-L-prolyl-5-methyl-L-norleucyl-4-fluoro-L-phenylalanyl-L-phenylalaninamide (JPE-1375; JSM-1375);
- N-(3-Phenylpropionyl)-L-ornithyl-L-prolyl-3-cyclohexyl-D-alanyl-L-tryptophyl-L-arginine N-5.1-C-1.5-lactam (PMX-205);
- N,N'-Bis(4-amino-2-methylquinolin-6-yl)urea (NSC12155);
- N-[4-(Dimethylamino)benzyl]-N-(4-isopropylphenyl)-7-methoxy-1,2,3,4-tetrahydronaphthalene-1-carboxamide hydrochloride (W54011);
- L-Phenylalanyl-L-ornithyl-L-prolyl-3-cyclohexyl-D-alanyl-L-tryptophyl-L-arginine N-5.2-C-1.6-cyclic peptide;
- (4aR, 16aS)-6, 18- Dihydroxy-23(S)-[2(S)-hydroxy-2-[2(R)-hydroxy-6(R)-methyl-5(R)-[2(S)-methylbutyl]tetrahydro-2H-pyran-2-yl]propionamido]-22(S)-isopropyl-7(S),19(R)-dimethyldocosahydro-13H,22H-dipyridazino[6,[6,1-f:6',1'o][1,4,7,10,13,16]oxapentaazacyclononadecine-5,7,11,17,20,24-hexanone (L-156602).

5. A CSaR1 inhibitor for use according to any one of claims 1 to 3, wherein said inhibitor is an anti-C5aR1 antibody or aptamer selected from the group consisting of:
- Avdoralimab;
- MOR-044254;
- NOX-D20;
- Anti-C5aR1ab-C5-SiRNA conjugate, anti-C5aRlab-protamine-C5siRNA conjugate;
- m20/70 mlgG2a.1;
- 3C5;
- 7F3.

6. A C5aR1 inhibitor for use according to any one of claims 1 to 3, wherein said inhibitor is a C5aR1 non-competitive allosteric inhibitor selected from: (2R)-2-[3-(furan-2-carbonyl)phenyl]-N-[4-(trifluoromethyl)-1,3-thiazol-2-yl]propenamide (DF2427);
and
compounds having general formula (I): 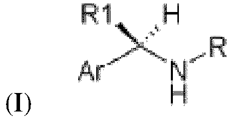 or a pharmaceutically acceptable salt thereof, wherein
**R** is selected from:
- 2-thiazolyl or 2-oxazolyl, unsubstituted or substituted by a group selected from methyl, tert-butyl or trifluoromethyl group;
- C(Ra)=N-W wherein W is linear or branched C₁-C₄ alkyl,
- CORa, SORa, SO₂Ra, PORa, PO₂Ra,
wherein **Ra** is selected from:
- C₁-C₅-alkyl, C₃-C₆-cycloalkyl, C₂-C₅-alkenyl, unsubstituted or substituted phenyl with a group selected from halogen, C₁-C₄-alkyl, C₁-C₄-alkoxy, halo-C₁-C₄-alkoxy, hydroxy, C₁-C₄-acyloxy, phenoxy, cyano, nitro, amino;
- a heteroaryl group selected from pyridine, pyrimidine, pyrrole, thiophene, furane, indole, thiazole, oxazole, such heteroaryl being unsubstituted or substituted with a group selected from halogen, C₁-C₄-alkyl, C₁-C₄-alkoxy, halo-C₁-C₄-alkoxy, hydroxy, C₁-C₄- acyloxy, phenoxy, cyano, nitro, amino;
- a α or β carboxyalkyl residue consisting of straight or branched C₁- C₆-alkyl, C₃-C₆-cycloalkyl, C₂-C₆-alkenyl, C₁-C₆-phenylalkyl, optionally substituted with a further carboxy (COOH) group;
- an ω-aminoalkylamino group of formula (II):
wherein in said 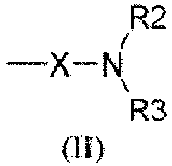 formula (II) X is selected from:
- linear or branched C₁-C₆ alkylene, C₄-C₆ alkenylene, C₄-C₆ alkynylene, optionally substituted by:
a) a CO₂R4 group, wherein R4 represents hydrogen or a linear or branched C₁-C₆ alkyl group or a linear or branched C₂-C₆ alkenyl group, or
b) a CONHR5 group wherein R5 represents
- hydrogen, linear or branched C₂-C₆ alkyl or an OR4 group, R4 being defined as above;
- a (CH₂)ₘ-B-(CH₂)ₙ, group, optionally substituted by a CO₂R4 or CONHR5 group, as defined above, wherein B is an oxygen, or sulfur atom, or nitrogen atom optionally substituted by a C₁-C₄ alkyl group, m is zero or an integer from 2 to 3 and n is an integer from 2 to 3; or B is a CO, SO or CONH group, m is an integer from 1 to 3 and n is an integer from 2 to 3;
**R2** and **R3** are independently selected form hydrogen, linear or branched C₁-C₆ alkyl, optionally interrupted by an oxygen or sulfur atom, a C₃-C₇ cycloalkyl, C₃-C₆ alkenyl, C₃-C₆-alkynyl, aryl-C₁-C₃-alkyl, hydroxy-C₂-C₃-alkyl group; or
**R2** and **R3** together with the N atom to which they are bound, form a 3-7 membered nitrogen heterocyclic ring of formula (III) 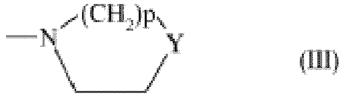 wherein **Y** represents:
- a single bond, CH₂, O, S, or a N-R6 group, where R6 represents hydrogen, C₁-C₄ alkyl, C₁-C₄ acyl, unsubstituted or substituted phenyl with a group selected from halogen, C₁-C₄-alkyl, C₁-C₄- alkoxy, hydroxy, C₁-C₄-acyloxy, phenoxy, cyano, nitro, amino, and p represents an integer from 0 to 3;
- a residue of formula SO₂R7 wherein R7 is C₁-C₆-alkyl, C₃-C₆-cycloalkyl, C₂-C₆-alkenyl, aryl and heteroaryl;
or
in said formula (II), **X** together with the nitrogen atom to which it is bound and with the **R2** group, forms a nitrogen containing 3-7 membered heterocyclic, monocyclic or polycyclic ring, and **R3** is selected from the group comprising hydrogen, C₁-C₄ alkyl, C₁-C₄ acyl, unsubstituted or substituted phenyl with a group selected from halogen, C₁-C₄-alkyl, C₁-C₄-alkoxy, hydroxy, C₁-C₄- acyloxy, phenoxy, cyano, nitro, amino;
**R1** is linear or branched C₁-C₅ alkyl, C₃-C₅ cycloalkyl; **Ar** is selected from:
- a phenyl group unsubstituted or substituted by one or more groups independently selected from halogen, C₁-C₄-alkyl, C₁-C₄-alkoxy, hydroxy, C₁-C₄-acyloxy, phenoxy, cyano, nitro, amino, C₁-C₄-acylamino, halo-C₁- C₃- alkyl, halo-C₁-C₃-alkoxy, benzoyl, heteroaryl carbonyl, heteroaryl, linear or branched C₁-C₈-alkanesulfonate, linear or branched C₁-C₈-alkanesulfonamides, linear or branched C₁-C₈ alkyl sulfonylmethyl; or
- a heteroaryl ring selected from pyridine, pyrrole, thiophene, furan, indole.

7. A C5aR1 inhibitor for use according to claim 6, wherein in said compound of formula (I)
**R** is selected from:
- 2-thiazolyl or 2-oxazolyl, unsubstituted or substituted by a group selected from methyl, tert-butyl or trifluoromethyl group,
- C(Ra)=N-W wherein W is linear or branched C₁-C₄ alkyl,
- CORa, SORa or SO₂Ra,
wherein Ra is as defined in claim 6; and
**Ar** is selected from the group comprising:
3'-benzoylphenyl, 3'-(4-chloro-benzoyl)-phenyl, 3'-(4-methylbenzoyl)-phenyl, 3'-acetyl-phenyl, 3'-propionyl-phenyl, 3'-isobutanoyl-phenyl, 4'-isobutyl-phenyl, 4'-trifluoromethanesulfonyloxy-phenyl, 4'-benzenesulfonyloxy-phenyl, 4'-trifluoromethanesulfonylamino-phenyl, 4'-benzenesulfonylamino-phenyl, 4'-benzenesulfonylmethyl-phenyl, 4'-acetoxyphenyl, 4'-propionyloxy-phenyl, 4'-benzoyloxy-phenyl, 4'-acetylamino-phenyl, 4'-propionylamino-phenyl, 4'- benzoylamino-phenyl, 3' -(furan-2-carbonyl)-phenyl, 3'-(benzofuran-2-carbonyl)-phenyl, 3'-(thiophen-2-carbonyl)-phenyl, 3'-(pyridine-2-carbonyl)-phenyl, 3 '-(thiazole-2-carbonyl)-phenyl, 3'-(oxazole-2-carbonyl)-phenyl, 3'-(2-furyl)-phenyl, 3'-(2-oxazolyl)- phenyl, 3'-(3-isoxazolyl)-phenyl, 3'-(2-benzoxazolyl)-phenyl, 3'-(3- benzoisoxazolyl)-phenyl, 3'-(2-thiazolyl)-phenyl, 3'-(2-pyridyl)-phenyl, 3'-(2- thiophenyl)-phenyl;
or **Ar** is a heteroaryl ring selected from pyridine, pyrrole, thiophene, furan or indole.

8. A C5aR1 inhibitor for use according to claim 6 or 7, selected from:
- 4-{(1R)-1-[(phenylsulfonyl)amino]ethyl}phenyl trifluoromethanesulfonate;
- N-[(1R)-1-(3-benzoylphenyl)ethyl]benzenesulfonamide;
- 4-{(1R)-1-[(pyridine-3-ylsulfonyl)amino]ethyl}phenyltrifluoromethanesulfonate;
- N-[(1R)-1-(3-benzoylphenyl)ethyl]methanesulfonamide;
- N-{(1R)-1-[3-(2-furoyl)phenyl]ethyl}thiophene-2-sulfonamide;
- N-{(1R)-1-[3-(2-furoyl)phenyl]ethyl}methanesulfonamide;
- 4-{(1R)-1-[(thien-2-ylsulfonyl)amino]ethyl}phenyl trifluoromethanesulfonate;
- N-[(1R)-1 -(3 -benzoylphenyl)ethyl] thiophene-2-sulfonamide;
- N-[(1R)-1-(3-benzoylphenyl)ethyl]-3-pyrrolidin-1-ylpropane-1-sulfonamide;
- methyl 5-({[(1R)-1-(3-benzoylphenyl)ethyl]amino}sulfonyl)-2-furoate;
- 5-({[(1R)-1-(3-benzoylphenyl)ethyl]amino}sulfonyl)-2-furoicacid;
- 4-{(1R)-2-methyl-1-[(methylsulfonyl)amino]propyl}phenyltrifluoromethanesulfonate;
- N-((1R)-1-{4-[1-methyl-1-(phenylsulfonyl)ethyl]phenyl } ethyl)methanesulfonamide;
- 4-[(1R)-1-(isobutyrylamino)ethyl]phenyltrifluoromethanesulfonate;
- 4-{[(1R)-1-(pyridine-3-ylcarbonyl)amino]ethyl]}phenyltrifluoromethanesulfonate;
- N-[(1R)-1-(3-benzoylphenyl)ethyl]benzamide;
- N-[(1R)-1-(3-benzoylphenyl)ethyl]-2-furamide;
- N-[(1R)-1-(3-benzoylphenyl)ethyl]cyclobutanecarboxamide;
- N-[(1R)-1-(4-trifluoromethanesulfonyloxy)phenylethyl]-4-piperidin-1-yl butanamide (DF2593Y);
- 4-{(1R)-1-[(4-pyrrolidin-1-ylbutanoyl)amino]ethyl]}phenyl trifluoromethanesulfonate;
- 3-{(1R)-1-[4-(4-trifluoromethyl-1,3-thiazol-2-yl)amino]ethyl}phenyl) (phenyl)methanone;
- R(-)-2-[(4'-trifluoromethanesulfonyloxy)phenyl]-N-[3-(N'-pirrolidinyl)propyl] propionamide (DF2297X) or its chloride salt (DF2297A); and
- 5-[(1R)-1-(4-{[4-(trifluoromethyl)-1,3-thiazol-2-yl]amino}phenyl)ethyl]tetrazol-2-ide (DF3966Y) or its sodium salt (DF3966A).

9. A C5aR1 inhibitor for use according to any one of claims 6 to 8, selected from:
- N-[(1R)-1-(4-trifluoromethanesulfonyloxy)phenylethyl]-4-piperidin-1-yl butanamide (DF2593Y) and pharmaceutically acceptable salts thereof, preferably its chloride salt (DF2593A),
- R(-)-2-[(4'-trifluoromethanesulfonyloxy)phenyl]-N-[3-(N'-pirrolidinyl)propyl] propionamide (DF2297X) or its chloride salt (DF2297A), and
- 5-[(1R)-1-(4-{ [4-(trifluoromethyl)-1,3-thiazol-2-yl]amino}phenyl)ethyl]tetrazol-2-ide (DF3966Y) or its sodium salt (DF3966A).

10. A C5aR1 inhibitor for use according to claims 1-9, selected from: Avacopan, PMX-53, W54011, Avdoralimab, MOR-044254, PMX-205, DF2593A, DF2297A, DF2427 and DF3966A.

11. A C5aR1 inhibitor for use according to claims 6 to 10, which is DF3966A.

12. An ophthalmic composition comprising a therapeutically effective amount of a C5aR1 inhibitor as defined in claims 3-11 and at least one ophthalmologically acceptable excipient or carrier.
